# EUROPEAN PATENT APPLICATION

(11) **EP 0 633 004 A1**
(43) Date of publication of application: **11.01.1995**
(21) Application number: 94110539.7
(22) Date of filing: 06.07.1994
(51) Int. Cl.: A61B 19/02, A61B 17/32, A61B 5/14

(54) **Safety container for disposal of a lancet assembly**

(71) Applicant: APLS CO., LTD., Maniwa-gun, Okayama-ken (JP)
(72) Inventor: Morita, Susumi, Nishinomiya-shi, Hyogo-ken (JP)
(74) Representative: Kraus, Walter, Dr.

(57) **Abstract**

An assembly of a safety lancet device (10) and a safety container (12) which includes a lancet assembly (14) and a safety container for receiving and locking with the lancet assembly after pricking the skin. The lancet assembly has an outer surface and includes a lancet having a lance tip (44), an outer sleeve (16), and a lip (64) disposed along the outer surface of the assembly after the lancet assembly has been used to prick the skin. The container includes a cup-shaped body (80) having an inner surface defining an internal cavity with a closed end (82) and an open end (84) for receiving the lancet assembly. The container further includes an interlock (100) disposed along the inner surface of the container, protruding into the internal cavity. When the used lancet assembly is inserted into the container after use, the interlock engages the lip to prevent the lancet assembly from being removed from the container.

## Description

### FIELD OF THE INVENTION

The invention relates generally to finger pricking devices such as pricking devices, and more specifically to the disposal of such devices after use.

### BACKGROUND OF THE INVENTION

Various "finger-pricking" devices are commercially available to hospitals, clinics, doctors' offices, and the like, as well as to individual consumers. Such pricking devices include sharp-pointed lancets that are used to make a quick puncture or penetration of the patient's skin in order to provide a small outflow of blood. Various tests may be employed with only small amounts of blood so that blood flowing from a such a wound or puncture is normally sufficient for these tests.

After use, extra care must be taken by the user to avoid being punctured by a used lancet. The risks in handling used lancets are greatly increased due to present day concerns regarding communicable diseases transmitted through body fluids such as blood. The lancet must be carefully handled until it can be properly disposed. Advances have been made in recent years to increase safety in handling such used devices. For example, pricking devices are currently available which include lancet tip automatic retraction features, or single shot firing mechanisms. Further advances are necessary, however, to protect those persons who handle used lancets, such as health care workers.

One such lancet, for example, is shown in Danish Registered Design No. MR0933 (allowed on October 1, 1992). The Danish pricking device is a self-contained one which includes a unitary ejector and lancet assembly which is partially surrounded by a protective sheath or sleeve after use. The used lancet, however, still presents a danger of contamination in that it contains an aperture through which the lancet had protruded at one end of the sheath or sleeve where usually there remains a residue of the blood or body fluid produced by the prick.

In this regard, certain regulations may exist which govern the ultimate disposal of such devices in hospitals, clinics, doctors' offices, and the like. Such devices must be disposed in dedicated, disposal containers, commonly referred to as "Sharp's containers." Sharp's containers provide additional protection for individuals ultimately handling the disposed lancets. However, such containers are not always readily available at the particular location where lancets are utilized. Consequently, they must be transported to the site of the Sharp's container, prolonging the period during which the carrier could come into contact with any bodily fluids clinging to the device, and thereby increasing risk of infection for the health care workers.

These risks are equally applicable to pricking devices used in the home. Special care must be taken so that other individuals in the home, as well as those handling ultimate disposal of the garbage containing the used device, do not come in contact with the used device.

### OBJECTS OF THE INVENTION

It is a primary object of the invention to provide an assembly of a pricker device and a safety container that provides increased handling safety after use. A more specific object of the invention is to provide a safety container that mechanically interlocks with a used pricking device to prevent the device from thereafter being separated from the container and reused or in contact with any other individual.

Another object of the invention is to provide a container which cooperates with a self-contained pricker device as a packaging container prior to use, and as a cooperating depository for the device after use. It is a related object of the invention to provide a container that mechanically interlocks (for example engages) with the device after use, but cannot mechanically interlock with the device before use.

A further object of the invention is to provide a container that is inexpensive to manufacture and assemble.

An additional object of the invention is to provide a disposal device that is reliable and easy to use.

### BRIEF SUMMARY OF THE INVENTION

In accomplishing these and other objectives, the invention provides an assembly of a safety lancet assembly and a safety container which includes the lancet assembly and a safety container for receiving and locking with the lancet assembly after pricking the skin. The lancet assembly has an outer surface and includes a lancet having a lance tip such as a blade, an outer sleeve, and a lip disposed along the outer surface of the assembly after the lancet assembly has been used to prick the skin. The container includes a cup-shaped body having an inner surface defining an internal cavity with a closed end and an open end for receiving the lancet assembly. The container further includes an interlocking means disposed along the inner surface of the container, protruding into the internal cavity. When the used lancet assembly is inserted into the container after use, the interlock engages the lip to prevent the lancet assembly from being removed from the container. These and other features and advantages of the invention will be more readily apparent upon reading the following description of a preferred exemplified embodiment of the invention and upon reference to the accompanying drawings wherein:

### BRIEF DESCRIPTION OF THE DRAWINGS

Figure 1 is a perspective, exploded schematic view of an interlockable pricking device and a dedicated safety disposal container constructed in accordance with teachings of the invention.

FIG. 2 is a perspective schematic view of the pricking device prior to use disposed within the container of FIG. 1.

FIG. 3 is a cross-sectional schematic view of the pricking device and container taken along line 3-6 - 3-6 in FIG.2.

FIG. 4 is a cross-sectional schematic view of the pricking device in an intermediate position during use.

FIG. 5 is a cross-sectional schematic view of the pricking device showing the extended position of the lancet in phantom and the position after use in solid.

FIG. 6 is a cross-sectional schematic view of the pricking device disposed within the container after use.

FIG. 7 is enlarged schematic fragmentary of the interlocks of the container.

FIG. 8 is enlarged schematic fragmentary of the interlocks of an alternate embodiment of the container.

While the invention will be described in connection with certain preferred embodiments, there is no intent to limit it to those embodiments. On the contrary, the intent is to cover all alternatives, modifications, and equivalents included within the spirit and scope of the invention as defined by the appended claims.

### DESCRIPTION OF THE PREFERRED EMBODIMENT

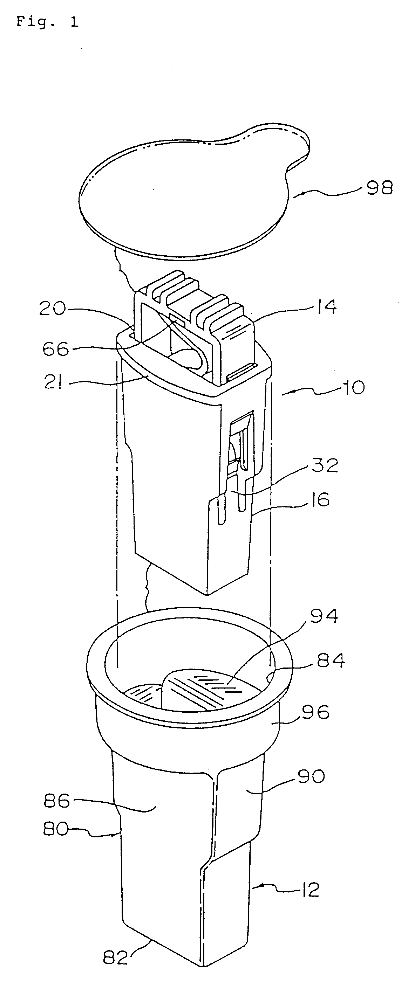

Turning now to the drawings, there is shown in FIG. 1 an exploded view of a pricking device 10, along with a container 12 constructed in accordance with teachings of the invention. While the invention will be described with reference to the particular pricking device illustrated, it will be appreciated that similarly constructed containers might well be utilized with alternate pricking devices. Such containers would necessarily take into consideration the particular design of the pricker utilized.

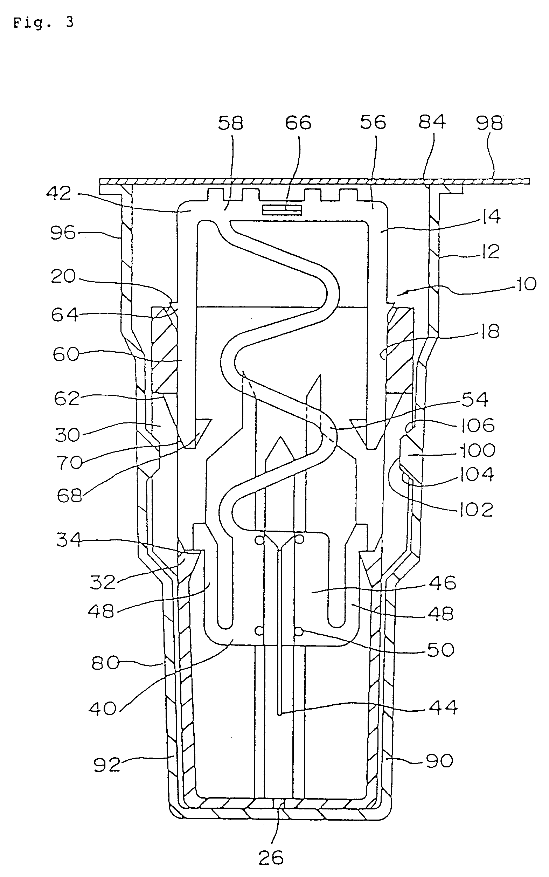

The pricking device 10 illustrated in the figures includes a unitary ejector and lancet assembly 14, and a sleeve 16. It is preferable that the components 14, 16 of the pricker device 10, excluding the lance tip 44, are made of a polymer, such as a polyacetal (POM) resin, a polybutylene terephalate resin or a polyester copolymer resin for the component 14 and an acrylonitrile-butadiene-styrene (ABS) resin, a polycarbonate resin or a polyester copolymer resin for the component 16, and are injection molded as unitary structures. During use, the components 14, 16 move between the relative positions shown in FIGS. 3, 4, 5, and 6.

The sheath or sleeve 16 includes a cavity 18 extending along an ejecting direction which cooperates with an opening 20 for receiving the unitary ejector and lancet assembly 14. Adjacent the opening 20 are outwardly extending flanges 21, shown in FIG. 1, which are used to hold the sleeve between the fingers of the user during operation of the device 10. The cavity 18 includes guidance channels (not shown) along opposite sides of the inner wall of the cavity 18, which cooperate with mating structures of the assembly 14 to control movement of the assembly within the cavity. The opposite end of the sleeve 16 is provided with an aperture 26 through which the lance tip 44 protrudes during use of the device 10.

The sleeve 16 further includes channels or openings 30 along opposite sides, which open into the cavity 18. For further controlling movement of the unitary ejector and lancet assembly within the sleeve, arms 32 disposed within the channels 30 include engaging extensions 34, which extend into the cavity 18. The significance of these components will become clear upon a more detailed explanation of the unitary ejector and lancet assembly 14.

The unitary ejector and lancet assembly 14 comprises a lancet 40 and an ejector 42. The lancet 40 includes a lance tip (or a needle tip) 44 and a lancet body 46 having cantilevered arms 48. To guide movement of the lancet 40 through the sleeve 16, pins 50 are provided along the remaining sides of the lancet body 46. As the assembly 14 is positioned within the sleeve 16 and actuated, the pins 50 cooperate with the guidance channels provided on the inner walls of the cavity 18 to control the periscoping movement of the lancet 40 within the sleeve 16. The lance tip 44, which is secured to and protrudes from one side of the lancet body 46, is formed from stainless steel or the like and includes a sharp point for piercing the patient's skin.

The ejector 42 includes a compressible spring 54 and a U-shaped actuator 56. The U-shaped actuator 56 includes a base portion 58, along which the compressible spring 54 is disposed, and upstanding actuator arms 60. The opposite end of the spring 54 is attached to the lancet body 46. The assembly 14 is sized such that it may be disposed and move within the opening 20 and cavity 18 of the sleeve 16 in the positions shown in FIGS. 3-6.

To retain the assembly 14 in position within the opening 20 of the sleeve 16 prior to actuation of the device 10, as shown in FIG. 3, outwardly extending lips 62, 64 are provided along the outer surfaces of the actuator arms 60. In this position, the lips 62 are disposed within the channels 30 such that they seat against the upper edge of each channel 30. The lips 64 are disposed along the open edge of the sleeve 16, adjacent the opening 20. It may be noted that the outer surfaces of the lips 64 are tapered from the opening 20 so that the U-shaped actuator 56 easily slides into the sleeve 16 upon depression of the actuator 56.

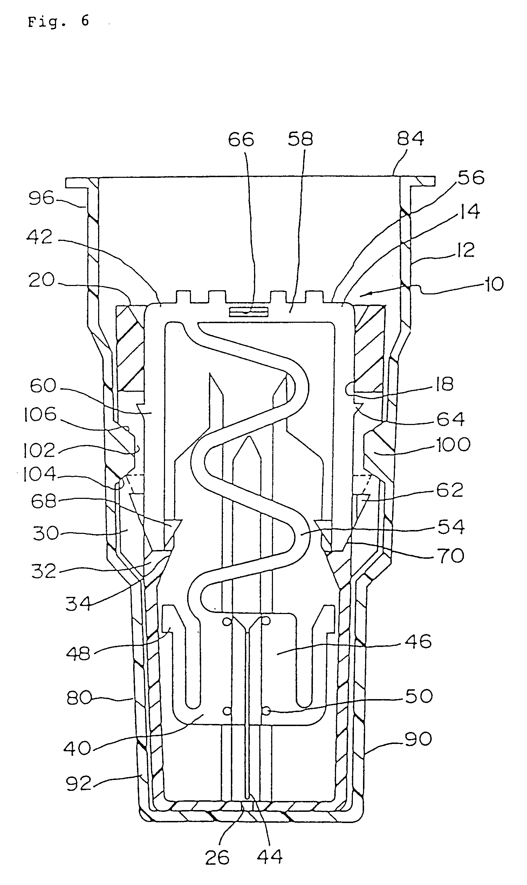

The lips 64 further function to lock the assembly 14 in position within the sleeve 16 after actuation of the device 10. As shown in FIG. 6, after use, the lips 64 are disposed within the channels 30 such that they may seat against the edge of each channel 30 and prevent the components 14, 16 from separating (substantially the same position previously occupied by the lips 62 in the unactuated position).

To further assist in maintaining the assembly 14 within the sleeve 16 after use, protrusions 66 extend outward from the side surfaces of the base portion 58, as shown in FIG. 1. After use, these protrusions 66 are disposed substantially adjacent the inner surface of the cavity to reduce movement of the assembly 14 within the sleeve 16.

The actuator arms 60 function as an actuator for releasing the lancet 40 to permit the lance tip 44 to protrude from the aperture 26, as will be apparent from the explanation set forth below. The ends of the actuator arms 60 are provided with a tapered lip 68 along the inner surface of the arms 60, and tapered surfaces 70 along the outside to the lips 62.

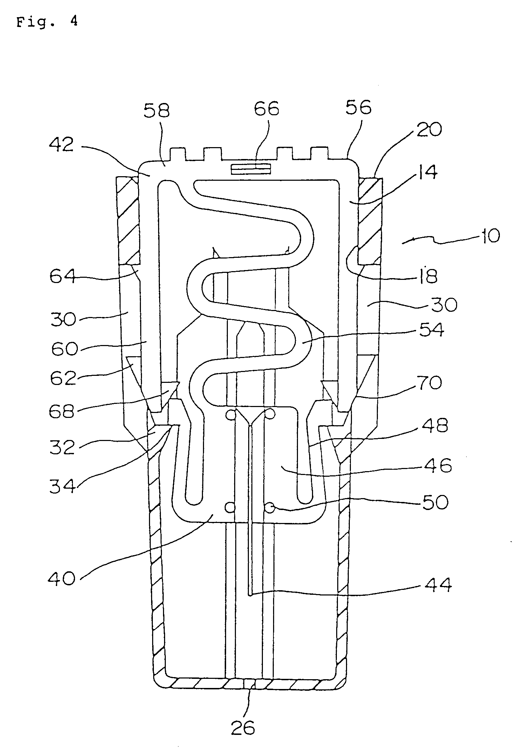

The operation of the device 10 may be described with reference to FIGS. 3-6. Prior to actuation, the components 14, 16 of the device 10 are disposed in the relative positions shown in FIG. 3. To use the device, sleeve 16 is taken between the fingers of the user and the end of the sleeve 16 containing the aperture 26 is placed against the skin of the patient. The user then uses the thumb to depress the U-shaped actuator 56 into the sleeve 16, as shown in FIG. 4. As the actuator 56 is depressed, the engaging extensions 34 of the sleeve 16 contact the cantilevered arms 48 of the lancet 40 to hold the lancet 40 stationary and compress and energize the spring 54.

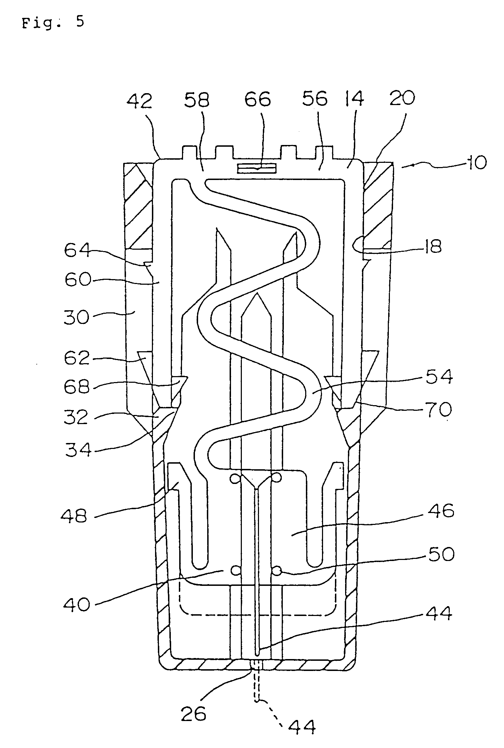

As the user continues to depress the actuator 56, the ends of the actuator arms 60 contact and separate the engaging extensions 34 of the sleeve 16 from the cantilevered arms 48 of the lancet 40. As the extensions 34 and the arms 48 separate, the force of the compressed spring 54 over comes the retaining force, and the lancet 40 is released. The compressed spring 54 then extends to the position shown in phantom in FIG. 5 and propels the lancet 40 forward, so that the lance tip 44 protrudes from the aperture 26 to pierce the skin. After extension, the spring 54 returns to its free-state position, as shown in solid lines in FIG. 5.

In accordance with the invention, there is provided a container 12 that cooperates with a pricking device to function as a package prior to use and interlocks with the pricking device after use in an assembly to enhance safety in handling the used device. According to an important aspect of the invention, the container mechanically engages the used pricking device and interlocks to prevent the device from being separated from the container and reused.

The container may be made of any suitable material. Generally, the container is preferably made of a polymer material such as a polyethylene or a polypropylene, from viewpoints of its production ease, cost and strength as a container.

As shown in FIG. 1, the container 12 preferably has a cup-shaped body 80 with a closed end 82 and an open end 84. Four walls 86, 90, 92, which descend from the open end 84, have inner surfaces that substantially conform to the outer surfaces of the sleeve 16. To accommodate the flange 21 of the sleeve 16, flats 94 extend outward from the walls 86 to an annular wall 96 adjacent the open end 84 of the container.

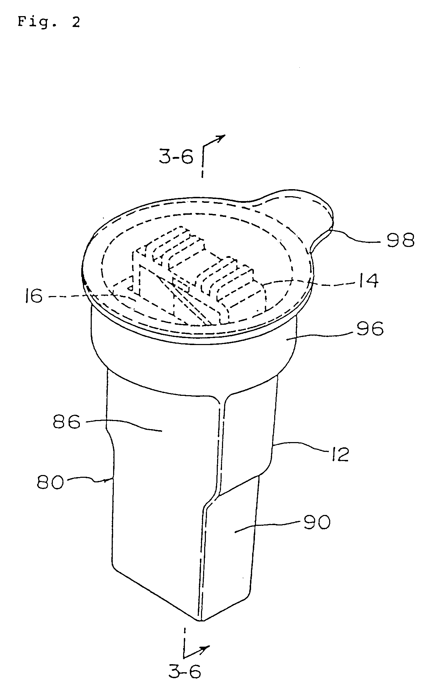

Before use, the pricking device 10 is disposed within the container 12, as shown in FIGS. 2 and 3, and sterilized. To maintain the sterility of the device 10, the device 10 is provided to the user in an airtight package. To hermetically seal the container 12, a sterilized paper 98 is sealed along the edge of the open end 84 of the container 12, as shown in FIGS. 1 and 2. In FIGS. 1-3, the sterilized paper 98 shown as a transparent material, however, it may be made of any appropriate material and sealed to the container 12 by any appropriate method. The sterilize paper 98 is made of any suitable material and sealed to the container 12 by any suitable manner. Moreover, the device may be very easily inserted into the container 12 on the production line, thereby providing a device that is suitable for high speed assembly lines.

The device 10 is provided to the user as shown in FIG. 2. The user simply removes the sterilized paper 98 to reveal the U-shaped actuator 56 and the flanges 21. To use the device, the user removes the device 10 from the container 12 by lightly holding the container 12 in one hand and tapping the open end 84 of the container 12 in the palm of the other hand, or, alternately, by simply grasping the U-shaped actuator 56 between the thumb and forefinger and removing the device 10. The device 10 may then be used to pierce the skin in the manner set forth above.

After using the device 10, it may be reinserted into the container 12 for further disposal. In order to securely hold the device within the container 12, interlocks 100 are provided along the inner surfaces of the walls 90, 92 of the container 12.

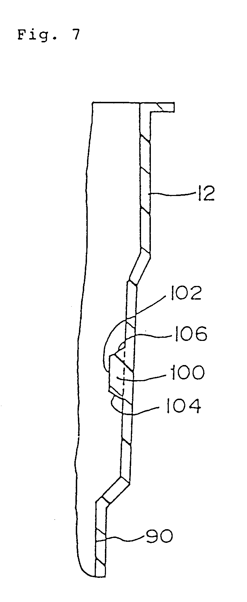

One embodiment of the interlocks 100 is illustrated in the enlarged fragmentary view shown in FIG. 7. The interlocks 100 include an elevated, substantially flat surface 102. Angled stops 104, 106 extend from the inner surfaces of the walls 90, 92 to the elevated surfaces 102 along opposite edges of the surface 102, while surfaces extend at right angles to the remaining edges of the surface 102. It will be appreciated that this design is particularly suited for injection molding.

When the used device is inserted into the container 12, the interlocks 100 and structure of the used device 10 engage to retain the device within the container 12. In the illustrated embodiment, insertion of the device into the container 12 disposes the interlocks 100 within the channels 30 of the sleeve 16, as shown in FIG. 6. In this position, the lips 62 of the unitary ejector and lancet assembly 14 of the used device 10 and the interlocks 100 of the container 12 engage to prevent the device 10 from separating from the container 12.

According to an important aspect of the invention, it is not possible for the device 10 to be restrained within the container 12 prior to use because the lips 62 and the interlocks 100 cannot engage. As shown in FIGS. 3 and 7, the device 10 sits in the same position within the container both before and after use. However, in the unused position, the lips 62 are disposed adjacent the upper edges of the channels 30. In this position, it is not possible for those lips 62 to engage the interlocks 100 of the container 12. Consequently, the device 10 cannot become secured to the container 12 until after use.

Moreover, due to the close relationship between the inner surface of the container 12 and the outer surface of the sleeve 16, it is not possible to accidentally actuate the device 10 while it is still disposed within the container 12. The lips 62 are simply unable to move past the interlocks 100. As a result, the device 10 cannot be inadvertently actuated during assembly or shipment once the device 10 is assembled into the container 12.

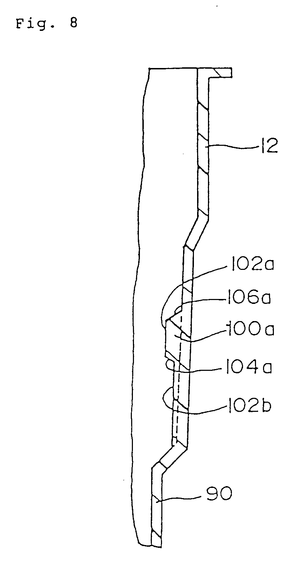

An alternate embodiment of the interlocks 100 is shown in FIG. 8. In this embodiment, the interlocks 100a have a stepped structure such that they include two flats 102a, 102b with the angled stop 104a disposed therebetween. This design is advantageous in that the flat 102a cooperates more closely with the outer surface of the sleeve 16, and, therefore, holds the pricker device 10 even more securely than a container 12 comprising interlocks constructed as shown in FIG. 7. Additionally, the embodiment shown in FIG. 8 can be more readily injection molded to provide a deeper interlock 100a than that shown in FIG. 7.

While it is preferred that the body 80 conform to the pricking device as shown and described, it will be appreciated that the body 80 could be of an alternate shape, so long as an adequate mechanical interlock is provided between the container 12 and the pricking device after actuation of the device. Moreover, the invention could be utilized with a self-contained disposable pricking device of an alternate design, or with a disposable "lancet and end cap" type of assembly used with a reusable base. It will be appreciated that after use the pricking device or lancet/end cap must present structure to which the interlock of the container may engage. Prior to actuation, however, the pricking device or lancet/end cap must not present such engaging structure.

It is to be understood that any allowed claims based on this application are to be accorded a range of equivalence commensurate in scope with the advance over the prior art.

## Claims

1. An assembly of a lancet assembly and a container comprising, in combination:
a lancet assembly for pricking skin, the lancet assembly having an outer surface and comprising
a lancet having a lance tip,
an outer sleeve, the lancet being substantially disposed within and passing through the outer sleeve to prick the skin,
a lip disposed along the outer surface after the lancet assembly has been used to prick the skin,
a safety container for receiving the lancet assembly after pricking the skin, the container having
a cup-shaped body having an inner surface defining an internal cavity with a closed end and an open end for receiving the lancet assembly, the body substantially surrounding the lancet assembly,
an interlock disposed along the inner surface protruding into the internal cavity, the interlock being disposed along the inner surface such that it engages the lip to prevent the lancet assembly from being removed from the container after the lancet assembly has been used to prick the skin has been inserted into the container.

2. The assembly as claimed in claim 1 wherein the interlock includes stops which angle to a raised surface.

3. The assembly as claimed in claim 1 wherein the lip is coupled to and moves with the lancet such that the lip assumes a first position prior to using the lancet assembly to prick the skin and a second position subsequent to using the lancet assembly to prick the skin.

4. The assembly as claimed in claim 3 wherein the sleeve includes a channel, the lip being disposed such that it moves within the channel.

5. The assembly as claimed in claim 4 wherein the interlock being disposed within the channel to engage the lip after the used lancet assembly is inserted into the container.

6. The assembly as claimed in claim 4 wherein the interlock includes stops which angle to a raised surface, the interlock being disposed within the channel such that at least one of the stops engages the lip after the used lancet assembly is inserted into the container.

7. The assembly as claimed in claim 1 wherein the interlock does not engage the lip when the lancet assembly is inserted into the container prior to use, whereby the container may be used as a shipping package prior to use of the pricking device and as a disposal container subsequent to use of the pricking device to pierce the skin.

8. The assembly as claimed in claim 5 wherein the interlock does not engage the lip when the lancet assembly is inserted into the container prior to use, whereby the container may be used as a shipping package prior to use of the pricking device and as a disposal container subsequent to use of the pricking device to pierce the skin.

9. The assembly as claimed in claim 6 wherein the interlock does not engage the lip when the lancet assembly is inserted into the container prior to use, whereby the container may be used as a shipping package prior to use of the pricking device and as a disposal container subsequent to use of the pricking device to pierce the skin.

10. The assembly as claimed in claim 7 further comprising a sealing paper for sealing against the open end of the container.

11. The assembly as claimed in claim 1 wherein the inner surface of the body substantially conforms to the outer surface of the lancet assembly.

12. The assembly as claimed in claim 1 wherein the interlock includes two substantially flat raised surfaces and a stop which angles between the surfaces.

13. A container for receiving a lancet assembly used for pricking skin, the lancet assembly having an outer surface, a lancet having a lance tip, an outer sleeve within which the lancet is substantially disposed and through which the lancet passes to prick the skin, and a lip which is disposed along the outer surface after the lancet assembly has been used to prick the skin, the safety container comprising, in combination:
a cup-shaped body having an inner surface defining an internal cavity with a closed end and an open end for receiving the lancet assembly, the body substantially surrounding the lancet assembly,
an interlock disposed along the inner surface protruding into the internal cavity, the interlock being disposed along the inner surface such that it engages the lip to prevent the lancet assembly from being removed from the container after the lancet assembly has been used to prick the skin and has been inserted into the container.

14. The container as claimed in claim 13 wherein the interlock includes stops which angle to a raised surface.

15. The container as claimed in claim 13 wherein the lip is coupled to and moves with the lancet such that the lip assumes a first position prior to using the lancet assembly to prick the skin and a second position subsequent to using the lancet assembly to prick the skin, the sleeve includes a channel, the lip being disposed such that it moves within the channel, and the interlock being disposed within the channel to engage the lip after the used lancet assembly is inserted into the container.

16. The container as claimed in claim 15 wherein the interlock includes stops which angle to a raised surface, the interlock being disposed within the channel such that at least one of the stops engages the lip after the used lancet assembly is inserted into the container.

17. The container as claimed in claim 13 wherein the interlock does not engage the lip when the lancet assembly is inserted into the container prior to use, whereby the container may be used as a shipping package prior to use of the pricking device and as a disposal container subsequent to use of the pricking device to pierce the skin.

18. The container as claimed in claim 17 further comprising a sealing paper for sealing against the open end of the container.

19. The container as claimed in claim 13 wherein the inner surface of the body substantially conforms to the outer surface of the lancet assembly.

20. The container as claimed in claim 13 wherein the interlock includes two substantially flat raised surfaces and a stop which angles between the surfaces.
